(19) Europäisches Patentamt / European Patent Office / Office européen des brevets

(11) **EP 3 405 897 B1**

(12) **EUROPEAN PATENT SPECIFICATION**

(45) Date of publication and mention
of the grant of the patent:
**04.12.2024 Bulletin 2024/49**

(51) International Patent Classification (IPC):
**G16B 20/20** (2019.01)   **G16B 50/10** (2019.01)
**G16B 40/30** (2019.01)

(21) Application number: **16707944.1**

(22) Date of filing: **18.01.2016**

(52) Cooperative Patent Classification (CPC):
**G16B 20/20; G16B 20/40**

(86) International application number:
**PCT/IB2016/000195**

(87) International publication number:
**WO 2017/125778 (27.07.2017 Gazette 2017/30)**

(54) **DETERMINING PHENOTYPE FROM GENOTYPE**

BESTIMMUNG DES PHÄNOTYPS AUS EINEM GENOTYP

DÉTERMINATION DU PHÉNOTYPE À PARTIR DU GÉNOTYPE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**28.11.2018 Bulletin 2018/48**

(73) Proprietor: **Outsee Limited
Cambridge CB4 2HY (GB)**

(72) Inventors:
 • **ZAUCHA, Jan**
  **Bristol BS8 2EW (GB)**
 • **THURLBY, Natalie**
  **Bristol BS8 3HN (GB)**
 • **GOUGH, Julian**
  **Bristol BS3 1 DP (GB)**

(74) Representative: **Appleyard Lees IP LLP
15 Clare Road
Halifax HX1 2HY (GB)**

(56) References cited:
**WO-A1-2013/044354    AU-A1- 2015 206 538
US-A1- 2016 048 634**

 • JIANGWEN SUN: "Machine Learning Approaches for Phenotype Refinement to Improve Genetic Association Analysis Recommended Citation Sun, Jiangwen, "Machine Learning Approaches for Phenotype Refinement to Improve Genetic Association Analysis", 1 January 2015 (2015-01-01), XP055301038, Retrieved from the Internet <URL:http://digitalcommons.uconn.edu/cgi/viewcontent.cgi?article=7042&context=dissertations>
 • ULRIKE VON LUXBURG: "A tutorial on spectral clustering", STATISTICS AND COMPUTING, KLUWER ACADEMIC PUBLISHERS, BO, vol. 17, no. 4, 22 August 2007 (2007-08-22), pages 395 - 416, XP019533997, ISSN: 1573-1375, DOI: 10.1007/S11222-007-9033-Z
 • SUN JIANGWEN ET AL: "Multi-view biclustering for genotype-phenotype association studies of complex diseases", 2013 IEEE INTERNATIONAL CONFERENCE ON BIOINFORMATICS AND BIOMEDICINE, IEEE, 18 December 2013 (2013-12-18), pages 316 - 321, XP032562269, [retrieved on 20140204], DOI: 10.1109/BIBM.2013.6732509

EP 3 405 897 B1

- SHIHAB H A ET AL: "Predicting the Functional, Molecular, and Phenotypic Consequences of Amino Acid Substitutions using Hidden Markov Models", HUMAN MUTATION, JOHN WILEY & SONS, INC, US, vol. 34, no. 1, 1 January 2013 (2013-01-01), pages 57 - 65, XP002761644, ISSN: 1059-7794, [retrieved on 20121102], DOI: 10.1002/HUMU.22225
- Y. LIU ET AL: "Analysis of correlated mutations in HIV-1 protease using spectral clustering", BIOINFORMATICS., vol. 24, no. 10, 28 March 2008 (2008-03-28), GB, pages 1243 - 1250, XP055301258, ISSN: 1367-4803, DOI: 10.1093/bioinformatics/btn110
- H. FANG ET AL: "dcGO: database of domain-centric ontologies on functions, phenotypes, diseases and more", NUCLEIC ACIDS RESEARCH, vol. 41, no. D1, 17 November 2012 (2012-11-17), GB, pages D536 - D544, XP055301326, ISSN: 0305-1048, DOI: 10.1093/nar/gks1080

- SHIHAB, H.A.; GOUGH, J.; COOPER, D.N.; BARKER, G.L.A.; EDWARDS, K.J.; DAY, I.N.M.; GAUNT, T.: "Predicting the Functional, Molecular, and Phenotypic Consequences of Amino Acid Substitutions using Hidden Markov Models", HUMAN MUTATION, vol. 34, no. 1, 2012, pages 57 - 65, XP002761644

## Description

### FIELD OF THE DISCLOSURE

The present disclosure is generally directed towards the prediction of the phenotypic characteristics of an individual, of any species, based on their genome, which contains genetic outliers within a population.

### BACKGROUND

Analysis of the deoxyribonucleic acid (DNA) has provided a wealth of information related to a particular species, population, pool of organisms, and even specific individuals. Continuous discoveries lead to the ever-increasing knowledge of genetic data and how that data impact an individual's phenotype. For example, if a subject carries a mutation in the gene MCM6, encoding the lactase protein, it fails to produce sufficient lactase and exhibits lactose intolerance.

There are numerous examples of genotype-phenotype relationships in humans in diverse areas including e.g., medical/health, fitness/sport, aptitudes, nutrition, physical/physiological characteristics, psychological/mental etc. phenotypes spanning the areas of medical/health, fitness/sport, aptitudes, nutrition, physical/physiological characteristics, psychological/mental, etc. These encompass susceptibility/risk (e.g., to injury or disease), rates (e.g., faster recovery or adaptiveness, metabolism), quantity (e.g., bone density, cardiac capacity), or binary (e.g., absence of wisdom teeth or presence of an enzyme), reaction (e.g., to drugs, alcohol, caffeine), etc. Many have been discussed in scientific publications and include information regarding the underlying variant. These are the "known variants" and, although significant, represent only a miniscule fraction of a person's genetic variation. There are three billion base pairs, of which the order of 100,000 variants vary per person in protein-coding exons alone (which constitute -1.5% of the human genome), and there exist only -175,000 "known variants" across all people (e.g., number of entries in HGMD the Human Gene Mutation Database).

### SUMMARY

The invention is defined by independent claim 1. It is with respect to the above issues and other problems that the embodiments presented herein were contemplated. As one embodiment and a general introduction, a subject individual of a species is selected for analysis. Predicting the phenotypic characteristics of an individual through a causal analysis via molecular biology approaches may result in the identification of the genetic determinants responsible. Embodiments of the present disclosure are generally directed towards linking the variant with the processes of phenotypic outlier detection with the identification of the genetic cause.

A determination is made to identify which relevant variants the subject has in view of the background population of the species. One may wish to choose a generic human background with as much diversity as possible or a more targeted background. For example, lactose intolerance may be a rarity (e.g., statistical outlier) for certain human subjects in a generic population but not, for example, when compared to a population sample of Chinese individuals. Performing an analysis to determine lactose intolerance in a Chinese subject against a generic background may be of little interest; therefore, a population sample of Chinese individuals may be selected. In this way, however, a different population background sample may be selected in order to determine the variants the subject has with respect to that population. The variants may be further addressed in terms of relevancy, such as the identification of an association between a variant and a phenotype or genetic trait and the strength of such a predictor in indicating the trait.

SUPERFAMILY is a database comprising a library of Hidden Markov Models (HMMs) that represent the structural classification of proteins (SCOP). SUPERFAMILY provides a mapping of structural domains of proteins encoding DNA sequences, including genomes and other sequence sets and is accessible via the Internet address "http://supfam.org/SUPERFAMILY/". Additional information on HMMs may be found in the paper "Predicting the Functional, Molecular, and Phenotypic Consequences of Amino Acid Substitutions using Hidden Markov Models." Human mutation 34(1), 57-65, Shihab, H.A., Gough, J., Cooper, D.N., Barker, G.L.A., Edwards, K.J., Day, I.N.M., Gaunt, T. (2012).

$D^2P^2$ is a database comprising records of pre-computed disordered predictions from completely sequenced genomes. $D^2P^2$ is accessible via the Internet address "http://d2p2.pro/".

FATHMM ("Functional Analysis through Hidden Markov Models") is a resource for predicting the function consequences of both coding variants (e.g., non-synonymous single nucleotide variants (nsSNVs)), and non-coding variants. FATHMM is accessible via the Internet address "http://fathmm.biocompute.org.uk/".

dcGO is a database of domain-centric ontologies on functions, phenotypes, diseases, and additional related information. dcGO is accessible via the Internet address "http://supfam.org/SUPERFAMILY/dcGO/". The corresponding publication is "dcGO: database of domain-centric ontologies on functions, phenotypes, diseases and more", Fang et al., Nucleic acids research, vol. 41, no. D1, 17 November 2012.

Further, "Analysis of correlated mutations in HIV-1 protease using spectral clustering", Liu et al., Bioinformatics, vol. 24, no. 10, 28 March 2008, discloses the determination of the cooperativity of mutation in viral resistance.

"Predicting the Functional, Molecular, and Phenotypic Consequences of Amino Acid Substitutions using Hidden Markov Models", Shibab et al., Human Mutation, vol. 34, no. 1, 2012, discloses the prediction of the functional effects of protein missense variants using a precomputed database.

Spectral clustering provides a grouping based on a number of dimensions. Spectral clustering, when used in image analysis segments an image into logical associations, such as areas of an image, using vectors of color/brightness, etc. (e.g., in a landscape: sky, sun, tree, field, lake). In terms of a human, to which the embodiments herein relate, a phenotype such as eye color should segment the population into the basic eye colors using vectors of alleles: brown, green, blue, hazel, or grey. Quantitative scores are output for each individual person/pixel based on the difference they exhibit from their local neighborhood as opposed to a global one. A global comparison will simply rank the person with the bluest eyes as the furthest outlier, whereas spectral clustering will identify the one rare person who has yellow eyes but lies in the center of the eye color landscape.

In another embodiment, outlier detection is provided by intrinsic dimensionality. Intrinsic dimensionality is used for local outlier detection as a method of reducing the genetic variants down to a combination of those variants that are most relevant to predicting the phenotype in question. Where spectral clustering uses all dimensions, the intrinsic dimensionality describes how many variables are needed to represent the phenotype. Both methods have been previously used in computer vision. A more detailed definition and an example of usage of intrinsic dimensionality may be found in, "Intrinsic Dimensional Outlier Detection in High-Dimensional Data," Jonathan von Brünken, Michael E. Houle, and Arthur Zimek, National Institute of Informatics, which is available at the Internet address "http://www.nii.ac.jp/TechReports/15-003E.pdf."

In one embodiment, a method is disclosed, comprising: accessing, by a processor, genomic data of a number of individual organisms comprising a subject and a background population; identifying, by the processor, a number of variant sites in the genomic data; for one of the number of variant sites: retrieving, by the processor, from a first data repository by the processor, a strength-of-effect associated with the one of the number of variant sites; retrieving, by the processor, from a second data repository by the processor, a degree of association with an ontological term associated with the one of the number of variant sites; and determining, by the processor, that one of the number of phenotypes is relevant upon the strength-of-effect in combination with a degree of association with the ontological term being above a threshold amount; comparing, by the processor, a degree of match between a number of data pairs, each data pair comprising one datum of genomic data for the relevant phenotype from substantially each combination formed by two of the number of individuals; and generating, by the processor, a report for each of the pairs.

In another embodiment, a system is disclosed comprising: a communication interface; a processor; and wherein the processor performs accessing, by a processor, genomic data of a number of individual organisms comprising a subject and a background population; identifying, by the processor, a number of variant sites in the genomic data; for one of the number of variant sites: retrieving, by the processor, from a first data repository by the processor, a strength-of-effect associated with the one of the number of variant sites; retrieving, by the processor, from a second data repository by the processor, a degree of association with an ontological term associated with the one of the number of variant sites; and determining, by the processor, that one of the number of phenotypes is relevant upon the strength-of-effect in combination with a degree of association with the ontological term being above a threshold amount; comparing, by the processor, a degree of match between a number of data pairs, each data pair comprising one datum of genomic data for the relevant phenotype from substantially each combination formed by two of the number of individuals; and generating, by the processor, a report for each of the pairs.

It should be appreciated that the processor may comprise one or more of a microprocessor, one or more processing cores, one or more servers/blades, distributed processor (e.g., "cloud"), and/or other computing devices operable to carry out computations, programmed method steps and/or may be implemented to perform method steps disclosed herein. The processor may be associated with a communication interface, such as a network interface operable to access data locally (e.g., a bus, backplane, etc.), remotely (e.g., intranet, Internet, etc.), and/or other communication interfaces. The processor may further comprise human input-output signals to enable human input devices (e.g., mouse, trackpad, keyboard, etc.) to provide instructions to the processor and/or human output devices (e.g., screen, terminal, monitor, printer, etc.) to provide human-readable output. The processor may access a data repository for the reading/writing of data and, optionally retrieval of computer instructions that when read by the processor cause the processor to perform the method steps disclosed herein. The data repository may be a storage device accessed locally (e.g., internal drive, registers, memory chips, etc.) to the processor and/or remotely (e.g., external drive, networked storage device, Intranet/"cloud" storage, etc.). As a result, a processor may access programming instructions and data to perform certain method steps disclosed herein and output the results to a storage device and/or output device for production of a human-readable output.

The phrases "at least one," "one or more," and "and/or" are open-ended expressions that are both conjunctive and disjunctive in operation. For example, each of the expressions "at least one of A, B and C," "at least one of A, B, or C," "one or more of A, B, and C," "one or more of A, B, or C," and "A, B, and/or C" means A alone, B alone, C alone, A and B together, A and C together, B and C together, or A, B and C together.

The term "a" or "an" entity refers to one or more of that entity. As such, the terms "a" (or "an"), "one or more," and "at least one" can be used interchangeably herein. It is also to be noted that the terms "comprising," "including," and "having" can be used interchangeably.

The term "automatic" and variations thereof, as used herein, refers to any process or operation done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material."

The term "computer-readable medium," as used herein, refers to any tangible storage that participates in providing instructions to a processor for execution. Such a medium may take many forms, including, but not limited to, non-volatile media, volatile media, and transmission media. Non-volatile media includes, for example, NVRAM, or magnetic or optical disks. Volatile media includes dynamic memory, such as main memory. Common forms of computer-readable media include, for example, a floppy disk, a flexible disk, hard disk, magnetic tape, or any other magnetic medium, magneto-optical medium, a CD-ROM, any other optical medium, punch cards, paper tape, any other physical medium with patterns of holes, a RAM, a PROM, an EPROM, a FLASH-EPROM, a solid-state medium like a memory card, any other memory chip or cartridge, or any other medium from which a computer can read. When the computer-readable media is configured as a database, it is to be understood that the database may be any type of database, such as relational, hierarchical, object-oriented, and/or the like. Accordingly, the disclosure is considered to include a tangible storage medium and prior art-recognized equivalents and successor media, in which the software implementations of the present disclosure are stored.

The terms "determine," "calculate," and "compute," and variations thereof, as used herein, are used interchangeably and include any type of methodology, process, mathematical operation, or technique.

The term "module," as used herein, refers to any known or later-developed hardware, software, firmware, artificial intelligence, fuzzy logic, or combination of hardware and software that is capable of performing the functionality associated with that element. Also, while the disclosure is described in terms of exemplary embodiments, it should be appreciated that other aspects of the disclosure can be separately claimed.

## BRIEF DESCRIPTION OF THE DRAWINGS

The present disclosure is described in conjunction with the appended figures:

Figs. 1A-1E depict a process in accordance with embodiments of the present disclosure;
Fig. 2 depicts a report in accordance with embodiments of the present disclosure;
Figs. 3A-3D depict a first table in accordance with embodiments of the present disclosure;
Fig. 4 depicts a second table in accordance with embodiments of the present disclosure;
Fig. 5 depicts a system in accordance with embodiments of the present disclosure; and
Fig. 6 depicts a data processing model in accordance with embodiments of the present disclosure.

## DETAILED DESCRIPTION

The ensuing description provides embodiments only and is not intended to limit the scope, applicability, or configuration of the claims. Rather, the ensuing description will provide those skilled in the art with an enabling description for implementing the embodiments.

Any reference in the description comprising an element number, without a subelement identifier when a subelement identifier exists in the figures, when used in the plural, is intended to reference any two or more elements with a like element number. When such a reference is made in the singular form, it is intended to reference one of the elements with the like element number without limitation to a specific one of the elements. Any explicit usage herein to the contrary or providing further qualification or identification shall take precedence.

Certain embodiments, including systems and methods, disclosed herein may be embodied as software, hardware, software-hardware combinations (firmware), modules, and/or associated analysis hardware. However, to avoid unnecessarily obscuring the present disclosure, the following description omits well-known structures, components, and devices that may be shown in block diagram form and are well known or are otherwise summarized.

For purposes of explanation, numerous details are set forth in order to provide a thorough understanding of the present disclosure. It should be appreciated, however, that the present disclosure may be practiced in a variety of ways beyond the specific details set forth herein.

The prior art associates phenotypes to genotypes via genome-wide association studies (GWAS). GWAS target a specific phenotype, such as a human disease. GWAS typically collect genotype data from a large pool of individuals, commonly 1000-10,000, some of which will and will not have the phenotype selected. Statistically significant correlations are then sought between a genetic feature and the disease.

Despite the successes of GWAS, limitations exist. One limitation is the massive scale of the required genotyping. Another limitation is the insufficiency of statistical power to associate combinations of genotypes with phenotypes. Still another limitation is that the associations of the genotypes are often non-causal correlations. Additionally, cohort size on statistical power results in only finding significant associations with common variants. The embodiments provided herein address these and other limitations and advance the state of the art of, at least, genotype-phenotype association determination. Benefits of certain embodiments provided herein allow causal relationships to be determined, require a smaller number of background samples compared to GWAS, provide associations for common or rare variants, detect combinatorial genotypes, and may be more reliably predictive.

In one embodiment, a single subject, such as a human, provides their genetic data as an input. The data may be complete or comprise only a portion of their complete genetic data. The data is then used to predict aspects of their phenotype that are unusual or outstanding when compared to a population, which may be a general population (e.g., humans) or more targeted (e.g., humans of Korean ancestry).

In one embodiment, systems and methods are described directed to the prediction of attributes of a variant. In another embodiment, multiple cross-referenced data sets are used and, when taken together, provide a statement about a variant that is not known in the body of scientific literature. Predictions may then be made about more variants than are "known." In a further embodiment, predictions may be made on rare variants, even to the point of being unique to one person. In another embodiment, predictions may be made on the combinations of multiple variants (i.e., if a person has variant "a" and "b," "a" and "b" but not "c," or not "d," etc.).

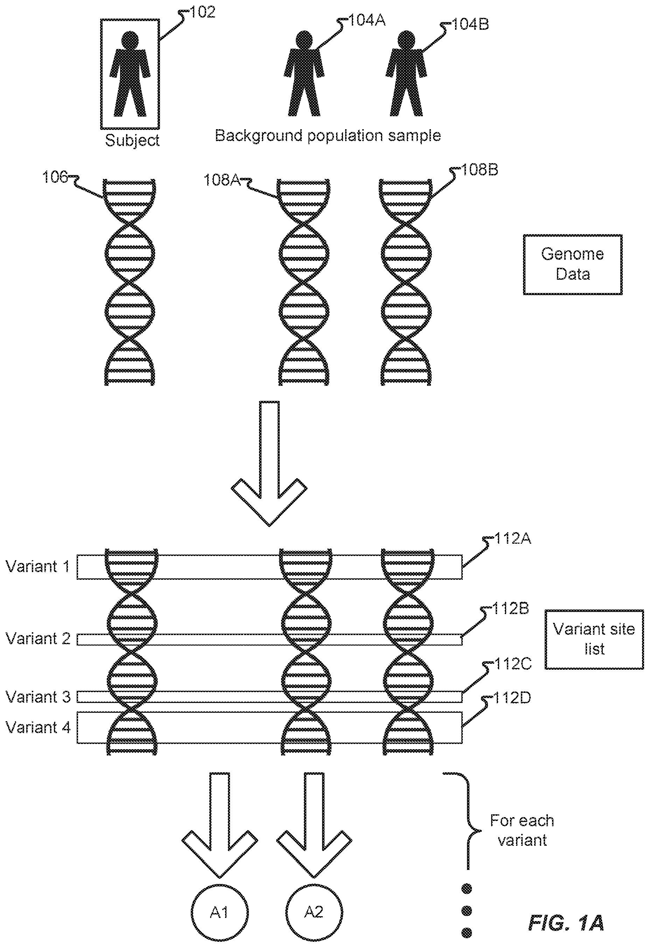

Figs. 1A-1E depicts a process in accordance with embodiments of the present disclosure. In one embodiment, a variant-effect predictor tool and a function-effect predictor tool are each provided with genotype data using protein domains of a background population and a subject. The results are combined, such as by utilizing spectral clustering, and optionally configured based upon selected parameters. The results may then be ranked and prioritized, such as to provide outlier details for a given phenotype, which may include a determination of a most extreme phenotype for a subject.

In one embodiment, genomic data 106, 108A-108B are gathered from a pool of organisms. The genotype data may be a collection of single nucleotide polymorphisms (SNPs), an exome, or a full genome sequence. Genomic data comprise genomic data 106 from at least one subject 102 and genomic data 108 from background population 104A-104B. Background population 104 is preferably selected to comprise a statistically significant number of members. Subject 102 may be, or not be, determined to be an outlier in the background population within a previously determined significance, as may be determined by an objective. For example, subject 102 may be a known outlier with respect to one phenotype for a background and a determination is desired as to other phenotypes for which the subject may, or may not be, an outlier. In another example, subject 102 has unknown outlier status. In yet another embodiment, background population 104 is selected to be arbitrarily large (e.g., 1000 members or more).

In another embodiment, the selection of substantially all members of background population 104 is selected in accordance with a desired determination against a desired population. For example, subject 102 has a number of known general characteristics (e.g., gender, ethnicity, etc.) and/or known specific characteristics (e.g., phenotypes). Background population 104 may then be selected having the same, different, or a mix of similar, general, and/or specific characteristics. For example, subject 102 is a Korean male and, if it is desired to determine the significant genetic outliers of subject 102 among all Korean males, then background population 104 preferably comprises only Korean males. However, if an object of a particular analysis is to determine the significant outliers of subject 102 compared to all humans, then background population 104 preferably comprises a representative sample of the entire human population. In another embodiment, background population 104 is selected from a public genome database (e.g., "23andme"). Genomic data 106, 108 may be complete or contain one or more portions of a complete genome.

In another embodiment, genomic data 106, 108 is then converted into a list of variant sites relative to a background population and processed into variant sites 112A-112D in Variant Call Format (VCF). VCF provides one way to store the genome of an individual organism in terms of the difference to a reference genome, or all differences between multiple individuals in a cohort and each other.

Variant sites 112 are then processed to predict the molecular entities of which the variant is a part, controls, or encodes. In one embodiment, processing of variant sites 112 is performed using hidden Markov Model (HMM) predictions. In another embodiment, SUPERFAMILY and $D^2P^2$ are applied to genome 106 of subject 102. In another embodiment, variants in non-coding regions are associated with proteins via their proximity to genes or by known and predicted features such as regulatory elements upstream and downstream of the genes. In another embodiment, variants in non-coding regions are associated with proteins via associations using high throughput datasets such as CAGE, DNAase hypersensitive sites (DHS), RNA-seq, and replication timing or chromatin exposure.

Variant sites 112 are illustrated as single 112B, 112C or neighboring multiple 112A, 112D bases as a matter of convenience. Variant sites 112 may include non-neighboring bases, alternatives (e.g., A and B or C), exclusions (e.g.,

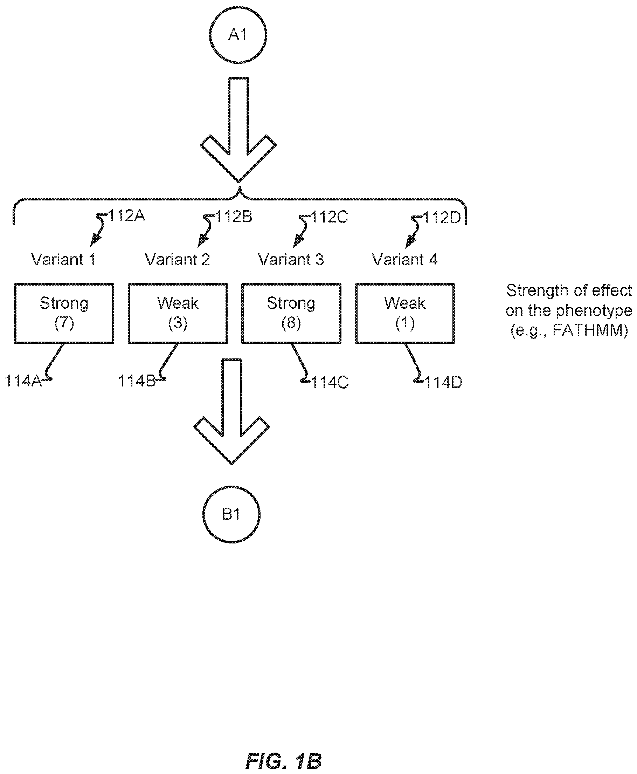

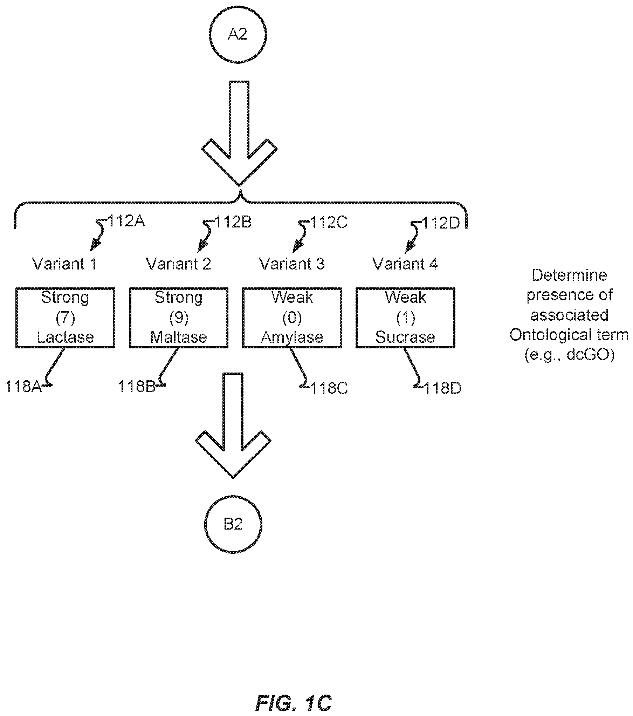

A and not D), and/or other variants that are or may become known. For each variant site 112, a strength effect is determined and ontological term identification is performed (see, Fig 1B and 1C, respectively).

Fig. 1B illustrates one embodiment wherein a strength-of-effect is determined for a phenotype for each variant site 112. In one embodiment, the variant-effect predictor tool comprises the FATHMM database. It should be appreciated that other strength-of-effect determinations may be made, such as by accessing other data repositories. It should also be noted that FATHMM may be untilized to make variant-effect predictions for both coding and non-coding variants and in the context of either somatic or germ-line mutations.

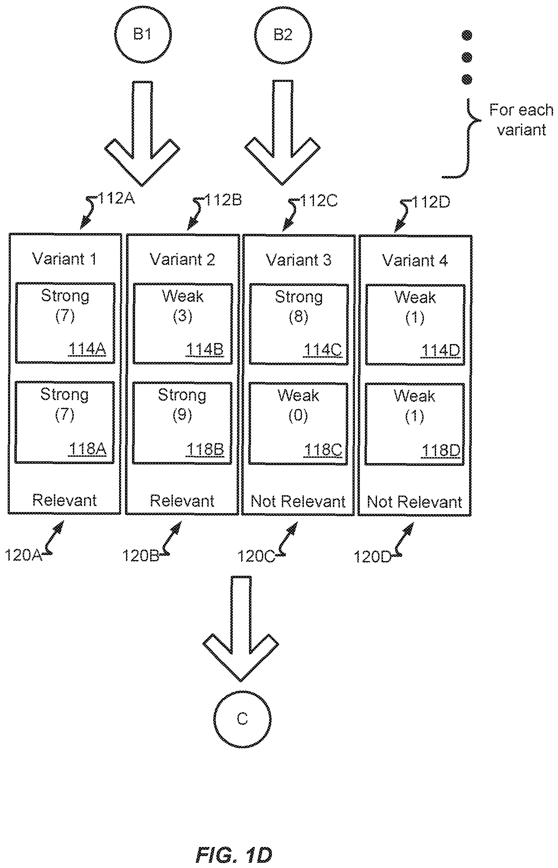

In one embodiment, each variant site 112 is compared to an entry in a database, or variant-effect predictor tool, accesses a causal relationship indicator, such as score 114A-114D. Score 114 may be a raw score, such as indicating the position of a particular variant (e.g., variant 112A) on a known scale. In another embodiment, other indicators or labels may be utilized, such as to readily identify variants that do and/or do not have a significant strength-of-effect to a phenotype (e.g., "strong," "weak," etc.) or as a matter of convenience. The resulting strength-of effect is aggregated with ontological term identification (see, Fig. 1D).

Fig. 1C illustrates one embodiment wherein a determination is made as to a variant (e.g., ones of variants 112) having an associated ontological term 118A-118D. For example, variant 1 (112A) may be associated with ontological term 118A (lactase). It should be appreciated that, due to variations within a single ontological term, the determination may be a score and a binary "yes/no" determination may indicate a determination score above/below a threshold value on a known scale. The resulting ontological term determination is then aggregated with strength-of-effect data (see, Fig. 1D).

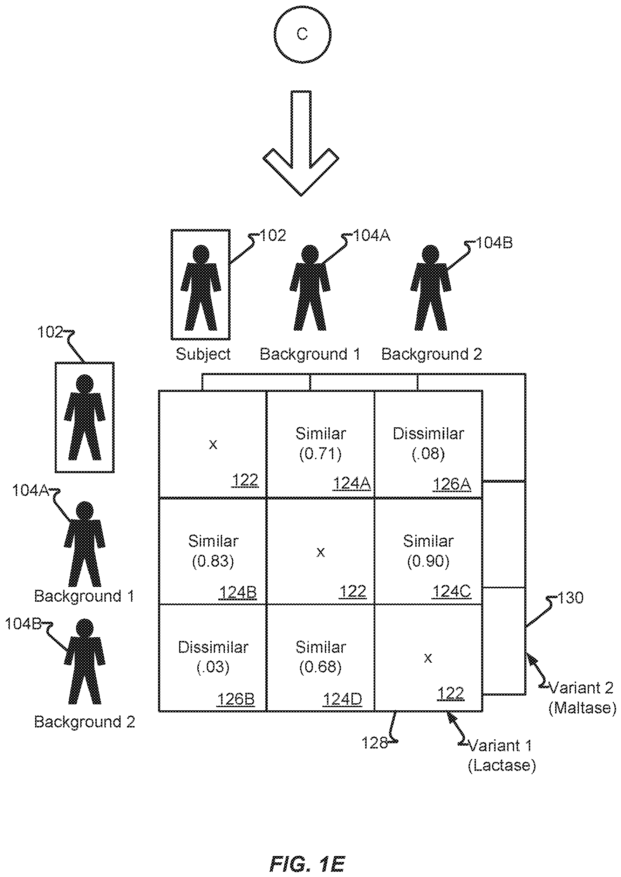

Fig. 1D illustrates one embodiment wherein a relevancy is determined as a combined strength-of-effect on a phenotype (see, Fig. 1B) and ontological term determination (see, Fig. 1C) to provide a single score indicating that at least one variant sites 112 is both a significant predictors and the significant predictor is of a known ontological term. Scores of one or both of strength-of-effect data and ontological term determination data may be weighted, such as to reflect a combination different from the sum of the individual data sources. In another embodiment, the scores may then be determined to be relevant 120A, 120B or not 120C, 120D, such as when the combined scores are above a previously determined threshold value or formula (e.g., top 0.5%, etc.). Variant sites 112 determined to be relevant data are then processed further (see, Fig. 1E).

Fig. 1E illustrates one embodiment, wherein distances are determined for subject 102 and background population 104 for each relevant variant (e.g., variant 1 (128), variant 2 (130), etc.). In one embodiment, matrices are formed for variant 128, 130. Cells 122 indicate same-to-same comparisons. Cells 124A-124D, 126A-126B may be scored to indicate a distance (or similarity, degree of match, etc.) between that particular organism (e.g., one of subject 102, background 104, etc.) and another organism (e.g., a different one of subject 102, background 104, etc.). Therefore, in one embodiment, each organism can be compared to each other organism. The distance between subject 102 and background 104 may then be indicated as an outlier, or not, for a particular variant.

In another embodiment, the score may be determined utilizing term frequency-inverse document frequency (TF-IDF).

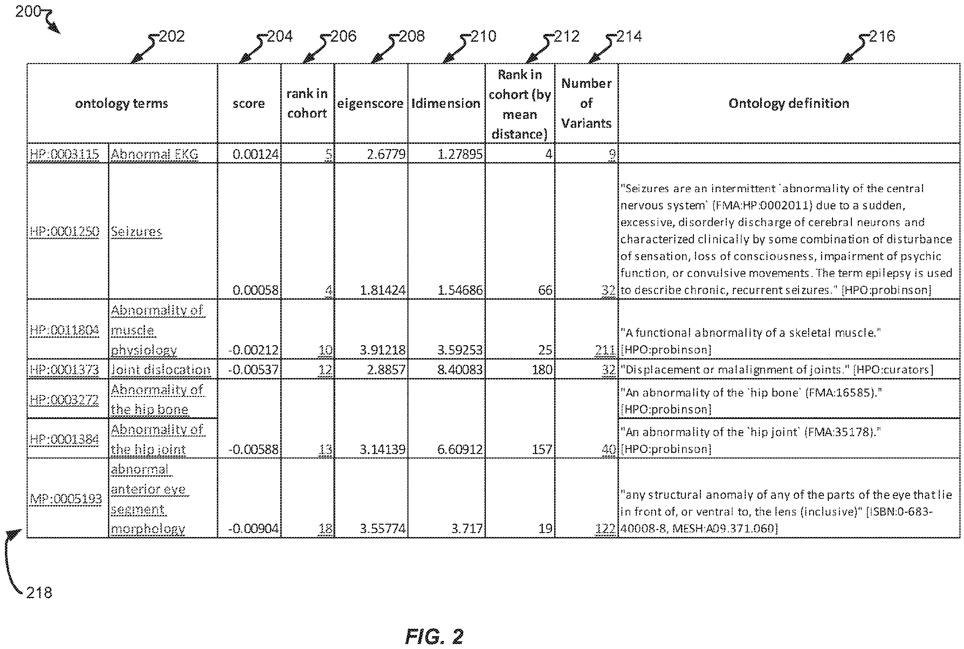

Fig. 2 depicts report 200 in accordance with embodiments of the present disclosure. Report 200 may be provided for an individual, such as subject 102 and may further provide variants, in rows 218 of table 200, having both an ontological term and a strength-of-effect above a threshold value and has been jointly determined, for one or more collections of variants relevant to the ontological term (e.g., number of variants 214 for a respective row 218), to have statistical differences from a background population. In one embodiment, three variants are individually low-scoring due to commonality within the population or at least a significant portion of the population (e.g., approximately half). However, if the combination of the three variants is rare, then such a combination may be highly significant. In another embodiment, spectral clustering is utilized to identify outliers comprising two or more variants.

Accordingly, report 200 may then comprise one or more rows 218 of ontological terms 202 (e.g., abnormal EKG), which in turn may comprise an identifier (e.g., "HP:0003115"), such as that taken from an ontology database such as "Human Phenotype" accessible via the Internet address http://human-phenotype-ontology.github.io, "Mammalian Phenotype," accessible via the Internet http://www.informatics.jax.org/searches/MP_form.shtml, "Gene Ontology," accessible via the Internet address http://amigo.geneontology.org, "Drugbank," accessible via the Internet address "http://www.drugbank.ca," and/or "dcGO" (see above) and/or other data repository of ontological terms.

In one embodiment, score 204 combines rank 206 and eigenscore 208. In another embodiment, score 204, combines rank 206 and idimension 210, in place of eigenscore 208. Score may be determined by formula:

$$score = (x * y * z)^{\frac{1}{3}}$$

$$(i)$$

wherein:

$$x = \frac{r^{1+\frac{rank}{n}}}{r}$$

(ii)

wherein:

$$y = \frac{eigenscore}{s}$$

(iii)

wherein:

$$z = \frac{eigenscore - m1}{m2}$$

(iv)

wherein:

$$r = e^{150}$$

(v)

wherein m1 is the minimum score of any individual in the cohort;
wherein m2 is the maximum score of any individual in the cohort;
wherein n is the number of individuals in the cohort;
wherein s is the sum of all Eigenscores in the cohort; and
wherein rank is rank within the background population.

In another embodiment, rank (e.g., rank in cohort 206) provides a ranking within the background population by score 204; eigenscore is eigenscore 208 indicating the raw score from spectral clustering; idimension 210, comprise the raw score from intrinsic dimensionality; rank in cohort by mean distance 212, comprise the rank within the background population by idimension 210; number of variants 214, comprise the number of variants that are relevant as determined by ontology association (e.g., as indicated by dcGO), and ontology definition 216, comprising the formal definition provided by the source ontology database.

As used herein, cohort comprises the participants (e.g., the background individuals, such as one or more background 104 and one or more subject 102) and provides the background plus the individual(s) in question. "Mean ave. distance" may be substituted for "Eigenscore," such as in score column 204, and optionally in formula (i), to report by intrinsic dimensionality instead of spectral clustering.

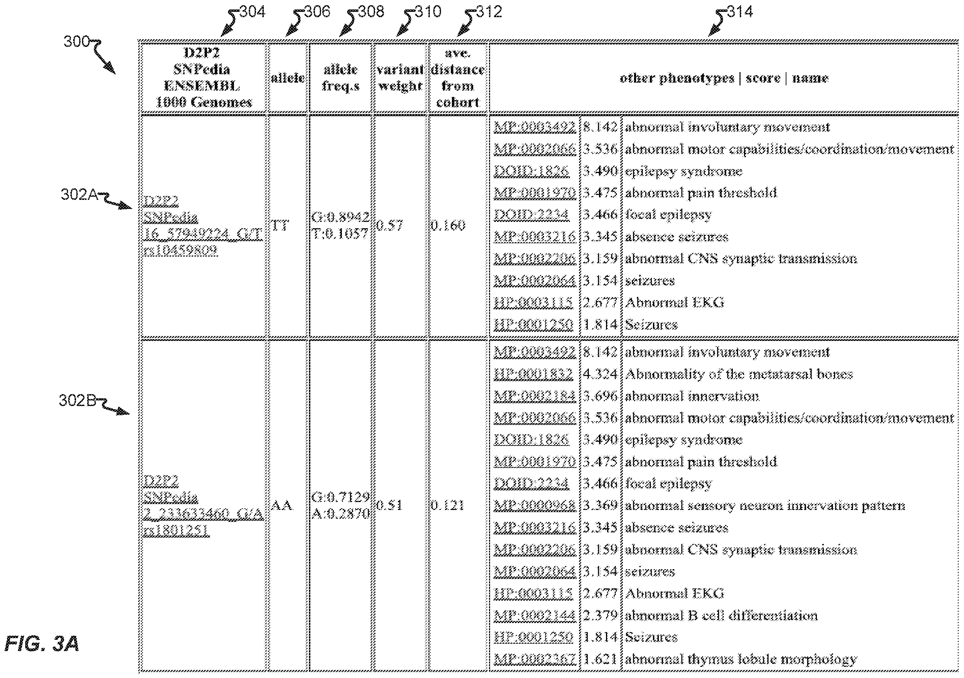

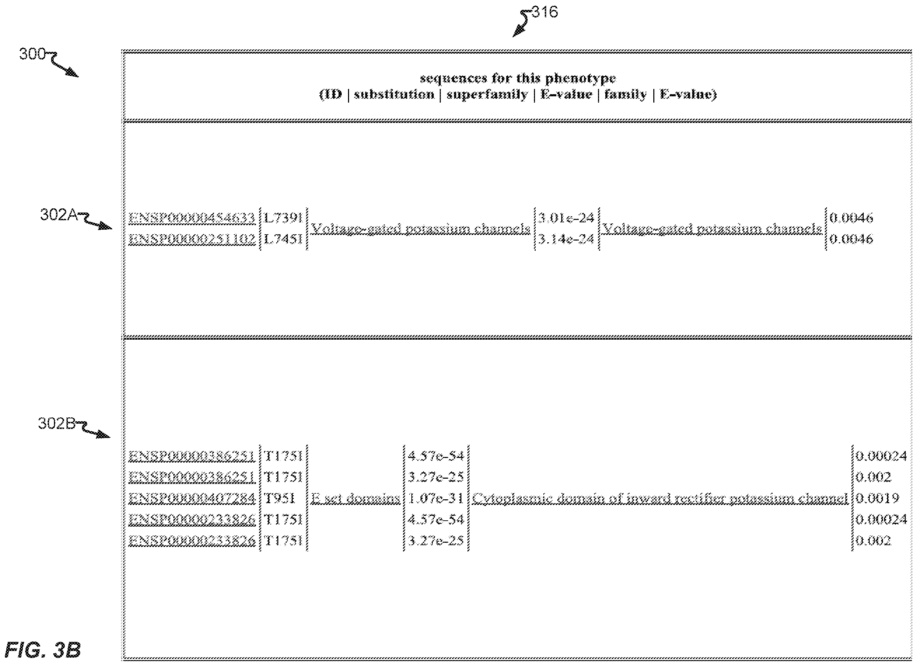

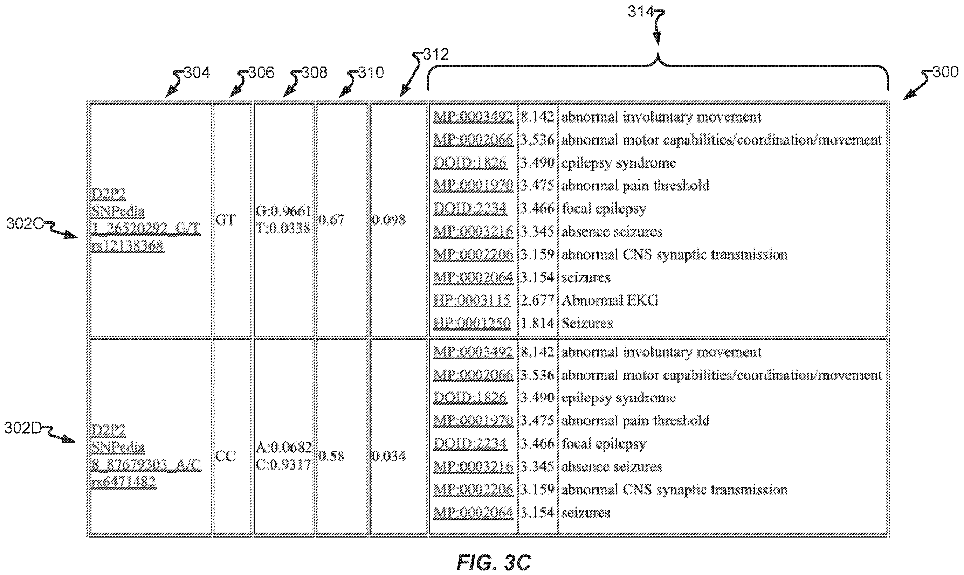

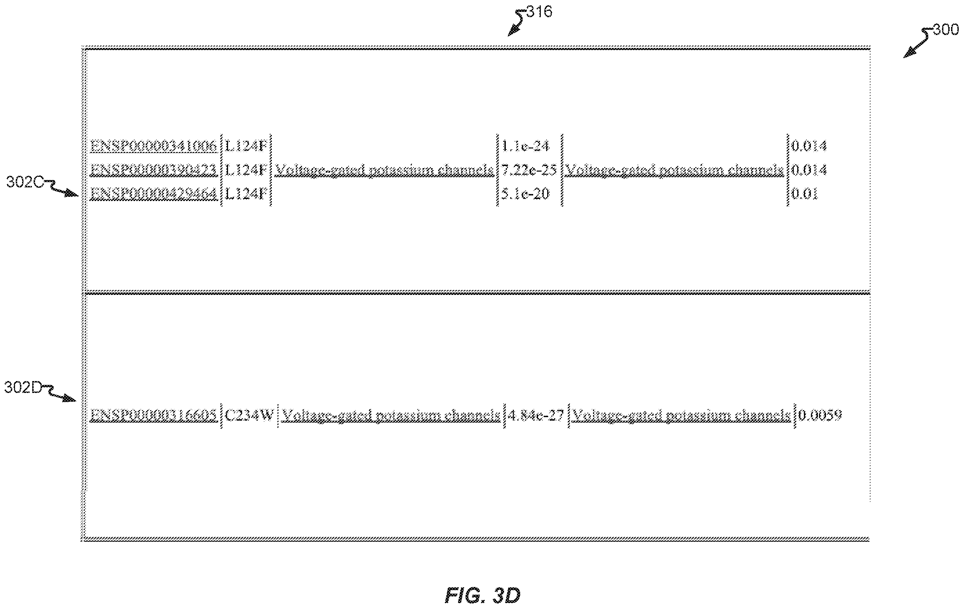

Figs. 3A-3D depict table 300 in accordance with embodiments of the present disclosure. In one embodiment, table 300 depicts rows 302A-302D and columns 304, 306, 308, 310, 312, 314, and 316. In one embodiment, table 300 presents the causal biological reason for the phenotype being an outlier. Table 300 corresponds to one row of table 200, i.e. column 214 lists only the number of variants whereas table 300 contains one row for each of those variants, e.g. for 'abnormal EKG' there are 9 variants and thus the complete table 300 would contain 9 rows, but only the first two are shown for illustration. Column 304 shows the genomic coordinates of the variant, column 306 shows the alleles of the subject, column 308 shows allele frequencies in the background, column 310 shows variant weight or consequence score (e.g., from FATHMM), column 312 shows average distance from cohort using the distance matrix (i.e., the contribution of that variant taken alone), column 314 identifies other phenotypes connected to the same variant, which may further include a score and name. Column 316 shows information about the variant (e.g., a corresponding one of row 302) namely sequences affected by the variant in a particular row 302 and may further comprise an identifier (ID), amino acid substitution, superfamily, e-value, family, and family e-value.

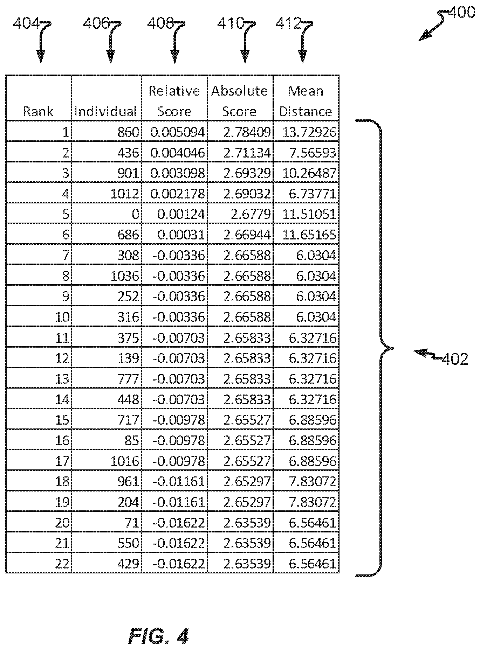

Fig. 4 depicts table 400 in accordance with embodiments of the present disclosure. In one embodiment, table 400 comprises a number of rows 402 and columns 404, 406, 408, 410, and 412. In one embodiment, table 400 can be used to see the distribution of scores amongst the background and to compare spectral clustering scores to intrinsic disorder scores. In one embodiment, column 404 provides a rank, for example, rank 206 of table 200 for one individual; entries in column 406 identify the individuals within row 402, such as by a numeric identifier. In another embodiment, an individual number of zero is associated with the subject such as subject 102; or, if more than one, one of a number of subjects 102. In another embodiment, column 408 shows a score relative to one individual (e.g. the subject in question in table 200), column 410 shows an absolute eigenscore as per column 208 for one individual, and column 412 provides the alternative score from intrinsic dimensionality as per column 210 for one individual.

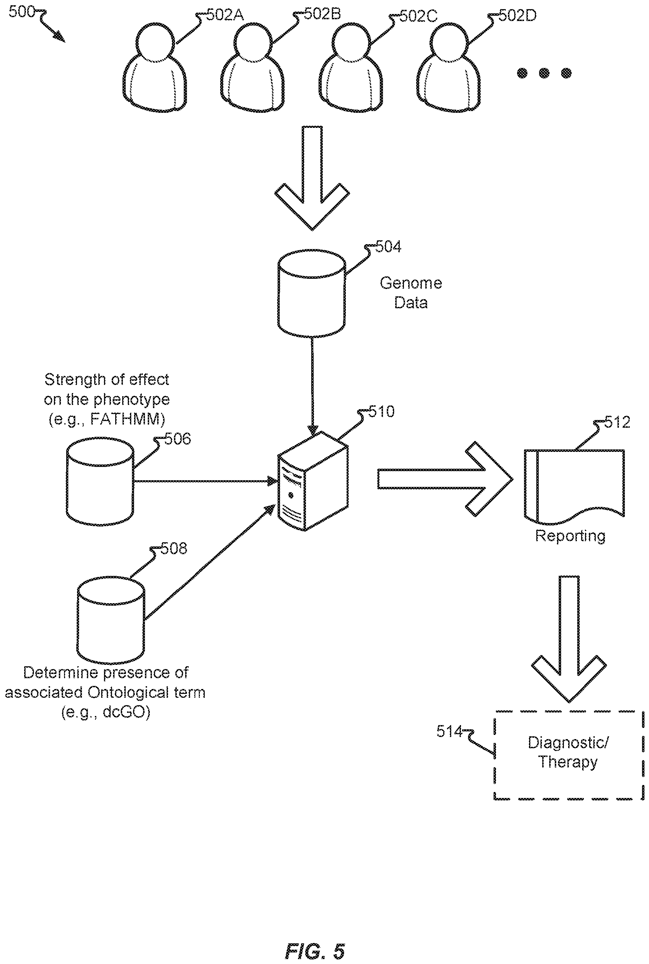

Fig. 5 depicts system 500 in accordance with embodiments of the present disclosure. In one embodiment, organisms 502A-502D provide genomic data 504, such as from individuals identified in column 406 (See, Fig. 4). At least one, but less than all organisms 502, is identified as a subject organism(s). Processor 510 accesses, for variant sites in genomic data 504, strength-of-effect data 506 and ontological term data 508 to determine significant outliers for a subject. Processor 510 may then provide report 512, such as ranking significant outliers, which may optionally be provided for diagnostic and/or therapeutic treatment 514.

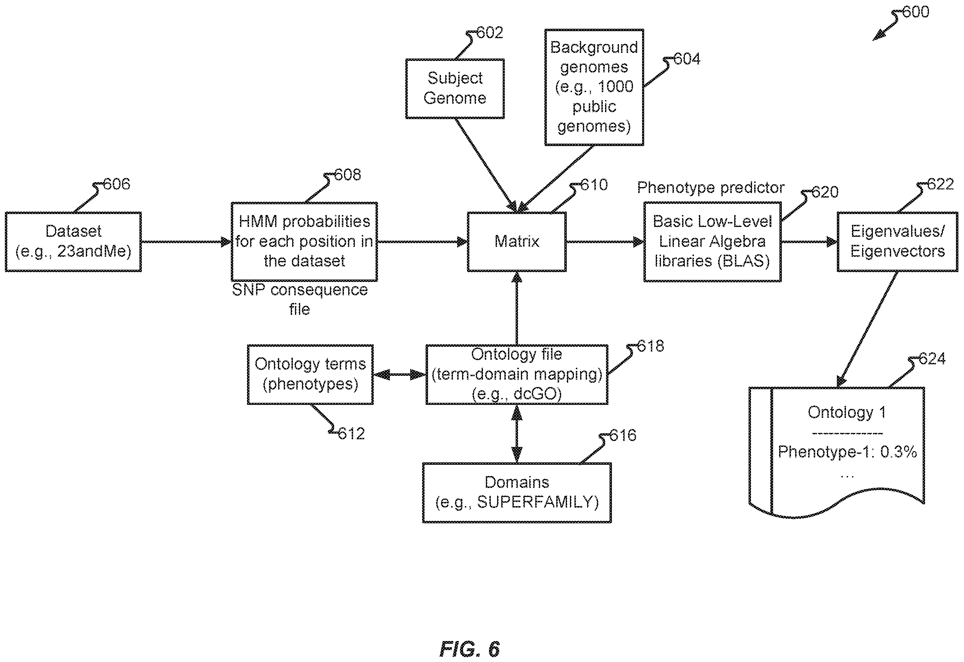

Fig. 6 depicts data processing model 600 in accordance with embodiments of the present disclosure. In one embodiment, subject genome 602 and background genomes 604 are combined in a matrix for each phenotype to determine distances between subject genome 602 and background genomes 604 for such phenotypes.

In one embodiment, a distance within matrix 610 is obtained between all participants (the combination of subject 102 and background population 104) by adding FATHMM distance contributions from each SNP mapped to the ontology term.

Next, the Gaussian kernel is determined by the formula:

$$G(x, \sigma) = \exp\left(-\frac{x^2}{\sigma^2}\right)$$

$$(\text{iv})$$

and is applied to transform the distance matrix into a similarity matrix, where

$$\sigma = \sqrt{\max{(x^2)}}.$$

$$(\text{iiv})$$

Then a modified version of spectral clustering is performed:
From the similarity matrix, the Laplacian (defined as Degree-Similarity) is obtained to solve the eigenproblem.

The second smallest eigenvector is used to partition the dataset into clusters. In another embodiment, a modified version of the elbow method is used to choose first N smallest eigenvectors to use for the clustering. Then the chosen eigenvectors are used to partition into clusters via standard K-means clustering.

Normally, when used for image partitioning, spectral clustering uses the first N eigenvectors to partition the image into N clusters. However, for genomic data, such as by utilization of multidimensional scaling, use of only the first eigenvector may be employed to partition the genomic data into more than two clusters. Two clusters may be particularly relevant in implementations partitioning on one SNP having all three options in common.

The number of clusters N may be chosen as described by Pham, Duc Truong, Stefan S. Dimov, and C. D. Nguyen. "Selection of K in K-means clustering." Proceedings of the Institution of Mechanical Engineers, Part C: Journal of Mechanical Engineering Science 219.1 (2005): 103-119.

Next, scores for each participant are calculated as follows:
The combination of the Euclidean distance from the center of the local cluster and the distance of the cluster from the global center.

A score is determined by the formula:

$$Score = Local\_score + \mu \cdot Global\_score$$

$$(\text{iix})$$

And the correction for cluster size is determined by the formula:

$$\mu = \frac{e^{\left(\gamma \cdot \frac{cohort\_size - cluster\_size}{cohort\_size}\right)} - 1}{e^{\gamma} - 1}$$

(ix)

Score comprises a distance between one of the genomic data and the rest of the background, as in column 208 of table 200. Local_score comprises an average Euclidian distance from one of the number of individual organisms to the cluster comprising each of the number of individual organisms in the same cluster as the subject.

Global_score comprises an average Euclidian distance for ones of the number of organisms within a cluster to all other of the number of organisms within the cohort.

Cohort_size comprises the number of the number of individual organisms in the cohort, which is the same as the number of rows as would be in the complete table, part of which is shown in figure 4.

Cluster size comprises the number of the number of individual organisms from the cohort in the cluster.

In another embodiment, dataset 606 is provided to SNP consequence file 608, such as comprising HMM probabilities. In another embodiment, ontological terms 612 are mapped via ontology file 618 (e.g., dcGO) to domains 616 (e.g., SUPERFAMILY). Ontology file 618 and SNP consequence file 608 may then be further utilized to determine distances within matrix 610.

In another embodiment, phenotype predictor 620, such as one using basic low-level linear algebra libraries (BLAS), produces eigenvalues and eigenvectors 622. As a result, report 624 may be produced, which may report the degree that subject genome 602 may be from background genomes 604 for a particular ontological term.

In another embodiment, variants may be prioritized, such as by prioritizing variants in the genome for further investigation. For example, in a cohort of patients with a known disease, variants may be prioritized on the basis of how common they are in the cohort and how rare in the background, such as in GWAS studies. In another embodiment, genes may be prioritized for further study in a manner similar to variant prioritization. It is possible that no two members of the cohort share the same variant, but they may all have different variants in a common gene, suggesting that gene should have a high priority.

In another embodiment, phenotype prioritization is provided, comprising prioritizing ontological terms for further investigation. An example would be for one individual, given all of their phenotype predictions may have priority given to those phenotypes for which that individual is a more or most of an outlier as compared to the background according to the genetic variation. There may be multiple genes/variants that contribute to the phenotype in one individual. Alternatively for a cohort with something in common, (e.g., a common disease), prioritization of phenotypes might identify which ontological terms have the most outlying genetic variation in associated genes within the cohort relative to a background.

In another embodiment, phenotype prioritization score comprises a score for each phenotype for an individual. The score may be the result of a function that takes into consideration both an absolute score, such as from spectral clustering or intrinsic dimensionality, and the distribution of such scores in the background, such as via ranking. To prioritize ontological terms for further investigation from an individual, the resulting prioritization score, such as obtained via formula (i), may be used to rank the phenotypes on priority. Phenotype prioritization may then identify the characteristics of an individual that are most genetically outlying with respect to the background. In a further embodiment, the result can be extended to phenotype prioritization of a cohort by choosing a threshold and, for each phenotype term, counting the number of members of the cohort who have an above-threshold score.

In another embodiment, gene and variant prioritization may be provided without phenotype prediction by examining the frequency of variation within a cohort versus a background. Another embodiment combines a variant score (e.g. from FATHMM) with the frequency. In such embodiments, gene and variant prioritization are taken from the phenotype prediction.

In another embodiment, there is no pre-conceived relevance of terms. For an individual, the variants are prioritized as a combination of the phenotype prioritization score and the variant prioritization score (e.g., the top priority variant will be that within the list of variants associated with the top-priority phenotype term), that has the top variant priority within that group. For gene prioritization the individual variant prioritization scores can be combined (e.g., via summation for each gene). For a cohort phenotype-based variant/gene prioritization may be similar to the forgoing but utilizing the cohort phenotype ranking instead of the individual. In another embodiment there is a pre-conceived notion of which phenotypes are of interest (e.g., in a cohort of neonatal fatalities), and the phenotype term (e.g., "neonatal lethal") may be used. A more general embodiments list terms associated with neonatal lethality (e.g., "developmental disorder," "abnormality of the placenta,") may be used. Variant or gene prioritization may be restricted to those ontological terms which may have some connection with the pre-conceived notion of relevance.

In yet another embodiment, a treatment may be applied to subject 102 based upon the results produced by embodiments disclosed herein. The treatment may be the application or avoidance of a drug, genetic treatment, surgery, exercise, physical/occupational therapy, diet, environmental, and/or other medical, pharmacological, genetic, or lifestyle treatment. Treatment may further comprise inclusion of atypical examinations in terms of type, frequency, or time of examination based upon, for example, a perceived susceptibility for a potential phenotype.

In the foregoing description, for the purposes of illustration, methods were described in a particular order. It should be appreciated that in alternate embodiments, the methods may be performed in a different order than that described. It should also be appreciated that the methods described above may be performed by hardware components or may be embodied in sequences of machine-executable instructions, which may be used to cause a machine, such as a general-purpose or special-purpose processor (GPU or CPU), or logic circuits programmed with the instructions to perform the methods (FPGA). These machine-executable instructions may be stored on one or more machine-readable mediums, such as CD-ROMs or other type of optical disks, floppy diskettes, ROMs, RAMs, EPROMs, EEPROMs, magnetic or optical cards, flash memory, or other types of machine-readable mediums suitable for storing electronic instructions. Alternatively, the methods may be performed by a combination of hardware and software.

Specific details were given in the description to provide a thorough understanding of the embodiments. However, it will be understood by one of ordinary skill in the art that the embodiments may be practiced without these specific details. For example, circuits may be shown in block diagrams in order not to obscure the embodiments in unnecessary detail. In other instances, well-known circuits, processes, algorithms, structures, and techniques may be shown without unnecessary detail in order to avoid obscuring the embodiments.

Also, it is noted that the embodiments were described as a process, which is depicted as a flowchart, a flow diagram, a data flow diagram, a structure diagram, or a block diagram. Although a flowchart may describe the operations as a sequential process, many of the operations can be performed in parallel or concurrently. In addition, the order of the operations may be re-arranged. A process is terminated when its operations are completed, but could have additional steps not included in the figure. A process may correspond to a method, a function, a procedure, a subroutine, a subprogram, etc. When a process corresponds to a function, its termination corresponds to a return of the function to the calling function or the main function.

Furthermore, embodiments may be implemented by hardware, software, firmware, middleware, microcode, hardware description languages, or any combination thereof. When implemented in software, firmware, middleware or microcode, the program code or code segments to perform the necessary tasks may be stored in a machine-readable medium, such as a storage medium. A processor(s) may perform the necessary tasks. A code segment may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

**Claims**

1. A method of predicting the phenotypic characteristics for an individual organism, comprising:

   accessing, by a processor, genomic data of a number of individual organisms comprising at least one subject and a background population;
   identifying, by the processor, a number of variant sites in the genomic data for the at least one subject in view of the background population;

   for each identified variant site:

   retrieving, by the processor, from a first data repository by the processor, a strength-of-effect on at least one phenotype associated with the identified variant site;
   retrieving, by the processor, from a second data repository by the processor, a degree of association with an ontological term associated with the identified variant site; and
   determining, by the processor, that the identified variant site is relevant when a weighted sum of a score for the strength-of-effect and a score for the degree of association with the ontological term is above a threshold amount;

   for each relevant variant site:

forming, by the processor, a matrix for the at least one phenotype, the matrix having a plurality of cells with each cell scored to indicate a distance comparing the degree of match between the at least one subject and each individual organism in the number of individual organisms to determine distances between the at least one subject and the background population for the at least one phenotype; and

generating, by the processor, a report for the at least one subject which includes relevant variants which have statistical differences from the background population by generating, by the processor, a spectral clustering score by

transforming the matrix into a similarity matrix using a Gaussian kernel G determined by the formula:

$$G(x, \sigma) = exp\left(\frac{-x^2}{\sigma^2}\right)$$

where

$$\sigma = \sqrt{\max(x^2)};$$

from the similarity matrix, solving an eigenproblem by obtaining the Laplacian to generate a plurality of eigenvectors;
selecting the first N smallest eigenvectors; and
using the selected eigenvectors to partition into clusters via standard K-means clustering.

2. The method of claim 1, wherein the at least one subject comprises a plurality of subjects.

3. The method of claim 1, wherein each distance in the matrix is obtained by adding all distribution contributions from each relevant variant from an associated repository entry for known consequences of variants, and wherein the associated repository is a Functional Analysis through Hidden Markov Model (FATHMM) database which predicts function consequences of non-synonymous single nucleotide variants and non-coding variants.

4. The method of claim 1, wherein the report further comprises an indicator of statistical significance for the at least one subject with respect to a plurality of phenotypes.

5. The method of claim 1, wherein the step of generating the report further comprises generating, by the processor, an intrinsic dimensionality score which describes how many variables are needed to represent the phenotype.

6. The method of claim 1, wherein the threshold amount is determined, in part, as a threshold variance indicating one relevancy score is an outlier derived from a collection of relevancy scores for each phenotype.

7. The method of claim 1, wherein the step of retrieving the strength-of-effect on a phenotype associated with the identified variant site, further comprises, retrieving the strength-of-effect from a Functional Analysis through Hidden Markov Model (FATHMM) database which predicts function consequences of non-synonymous single nucleotide variants and non-coding variants.

8. The method of claim 1,

wherein the report further comprises a phenotype prioritization score for each phenotype for the at least one subject;
the method further comprising:
ranking the phenotype prioritization scores.

9. The method of claim 8, further comprising extracting allele identification from a higher ranked phenotype in the ranked entries of the phenotypes.

10. The method of claim 1, wherein the distance is determined utilizing term frequency inverse document frequency, TF-IDF.

11. The method of claim 1, wherein:

a cohort comprises the at least one subject and the background population; and
the distance is determined by the formula:

$$Score = Local\_score + \mu \cdot Global\_score;$$

where, $\mu$ is a correction for cluster size for a cluster determined by the formula:

$$\mu = \frac{e^{\left(\gamma \frac{cohort\_size - cluster\_size}{cohort\_size}\right)} - 1}{e^{\gamma} - 1};$$

and
wherein score is a distance between genomic data of one individual organism and genomic data of the rest of the background population;
wherein the local score is an average Euclidian distance from the at least one subject to the cluster comprising the at least one subject;
wherein the global score is an average Euclidian distance for the number of individual organisms within the cluster to all other of the individual organisms within the cohort;
wherein the cohort size is the number of individual organisms in the cohort; and
wherein the cluster size is the number of individual organisms in the cluster.

12. The method of claim 1, wherein:

the at least one subject and the background population define a cohort; and
the report comprises a score is determined by the formula:

$$score = (x * y * z)^{\frac{1}{3}}$$

wherein:

$$x = \frac{r^{1 + \frac{rank}{n}}}{r};$$

wherein:

$$y = \frac{eigenscore}{s}$$

wherein:

$$z = \frac{eigenscore - m1}{m2}$$

wherein:

$$r = e^{150};$$

wherein ml is the minimum score of any individual organism in the cohort;
wherein m2 is the maximum score of any individual organism in the cohort;
wherein n is the number of individual organisms in the cohort;
wherein s is the sum of all eigenscores in the cohort;
wherein rank is the rank of the individual organism within the background population; and
wherein the eigenscore is the spectral clustering score.

**Patentansprüche**

1. Verfahren zum Vorhersagen der phänotypischen Eigenschaften eines individuellen Organismus, umfassend:

Zugreifen, durch einen Prozessor, auf genomische Daten einer Anzahl von individuellen Organismen, umfassend mindestens ein Subjekt und eine Hintergrundpopulation;
Identifizieren, durch den Prozessor, einer Anzahl von Variantenstellen in den genomischen Daten für das mindestens eine Subjekt unter Berücksichtigung der Hintergrundpopulation;
für jede identifizierte Variantenstelle:

Abrufen, durch den Prozessor, aus einem ersten Daten-Repository durch den Prozessor, einer Wirkungsstärke auf mindestens einen Phänotyp, der mit der identifizierten Variantenstelle assoziiert ist;
Abrufen, durch den Prozessor, aus einem zweiten Daten-Repository durch den Prozessor, eines Grads der Assoziation mit einem ontologischen Begriff, der mit der identifizierten Variantenstelle verknüpft ist; und
Bestimmen durch den Prozessor, dass die identifizierte Variantenstelle relevant ist, wenn eine gewichtete Summe eines Scores für die Wirkungsstärke und eines Scores für den Grad der Assoziation mit dem ontologischen Begriff über einem Schwellenwert liegt;
für jede relevante Variantenstelle:

Ausbilden, durch den Prozessor, einer Matrix für den mindestens einen Phänotyp, wobei die Matrix eine Vielzahl von Zellen aufweist, wobei für jede Zelle ein Score vorliegt, um einen Abstand anzugeben, der den Entsprechungsgrad zwischen dem mindestens einen Subjekt und jedem individuellen Organismus der Anzahl von individuellen Organismen vergleicht, um Abstände zwischen dem mindestens einen Subjekt und der Hintergrundpopulation für den mindestens einen Phänotyp zu bestimmen; und
Erzeugen, durch den Prozessor, eines Berichts für das mindestens eine Subjekt, welcher relevante Varianten enthält, die statistische Unterschiede zu der Hintergrundpopulation aufweisen, durch Erzeugen, durch den Prozessor, eines spektralen Clustering-Scores, durch Umwandeln der Matrix in eine Ähnlichkeitsmatrix unter Verwendung eines Gaußschen Kernels G, bestimmt durch die Formel:

$$G(x, \sigma) = exp\left(\frac{-x^2}{\sigma^2}\right),$$

wobei gilt

$$\sigma = \sqrt{max(x^2)};$$

aus der Ähnlichkeitsmatrix, Lösen eines Eigenproblems durch Erhalten des Laplace-Operators zum Erzeugen einer Vielzahl von Eigenvektoren;
Auswählen der ersten N kleinsten Eigenvektoren; und
Verwenden der ausgewählten Eigenvektoren zum Partitionieren der Cluster durch herkömmliches K-Means-Clustering.

2. Verfahren nach Anspruch 1, wobei das mindestens eine Subjekt eine Vielzahl von Subjekten umfasst.

3. Verfahren nach Anspruch 1, wobei jede Distanz in der Matrix durch Addition aller Verteilungsbeiträge jeder relevanten Variante aus einem damit verknüpften Repository-Eintrag für bekannte Folgen von Varianten erhalten wird und wobei das damit verknüpfte Repository eine FATHMM-Datenbank (Functional Analysis through Hidden Markov Model) ist, die Funktionskonsequenzen nicht-synonymer Einzelnukleotidvarianten und nichtkodierender Varianten vorhersagt.

4. Verfahren nach Anspruch 1, wobei der Bericht ferner eine Angabe der statistischen Signifikanz für das mindestens eine Subjekt in Bezug auf eine Vielzahl von Phänotypen umfasst.

5. Verfahren nach Anspruch 1, wobei der Schritt des Erzeugens des Berichts ferner das Erzeugen, durch den Prozessor, eines intrinsischen Dimensions-Scores umfasst, der beschreibt, wie viele Variablen zum Darstellen des Phänotyps erforderlich sind.

**6.** Verfahren nach Anspruch 1, wobei der Schwellenwert teilweise bestimmt wird als eine Schwellenwertvarianz, die angibt, dass ein Relevanz-Score ein Ausreißer ist, abgeleitet von einer Sammlung von Relevanz-Scores für jeden Phänotyp.

**7.** Verfahren nach Anspruch 1, wobei der Schritt des Erfassens der Wirkungsstärke auf einen Phänotyp, der mit der identifizierten Variantenstelle assoziiert ist, ferner das Erfassen der Wirkungsstärke aus einer FATHMM-Datenbank (Functional Analysis through Hidden Markov Model) umfasst, die Funktionsfolgen von nicht-synonymen Einzelnukleotidvarianten und nichtkodierenden Varianten voraussagt.

**8.** Verfahren nach Anspruch 1,

wobei der Bericht ferner einen Phänotyp-Priorisierungs-Score für jeden Phänotyp für das mindestens eine Subjekt umfasst;
das Verfahren ferner umfassend:
Einstufen der Phänotyp-Priorisierungs-Scores.

**9.** Verfahren nach Anspruch 8, ferner umfassend das Extrahieren einer Allelidentifikation von einem höher eingestuften Phänotyp in den eingestuften Einträgen der Phänotypen.

**10.** Verfahren nach Anspruch 1, wobei der Abstand bestimmt wird unter Einsatz von TF-IDF (term frequency - inverse document frequency).

**11.** Verfahren nach Anspruch 1, wobei:

eine Kohorte das mindestens eine Subjekt und die Hintergrundpopulation umfasst; und
der Abstand bestimmt wird unter Einsatz der Formel:

$$Score = Local\_score + \mu\ Global\_score;$$

wobei $\mu$ eine Korrektur in Bezug auf Clustergröße ist, für einen Cluster, bestimmt nach der Formel:

$$\mu\ =\ \frac{e^{\left(\gamma\frac{cohort\_size-cluster\_size}{cohort\_size}\right)}-1}{e^{\gamma}-1};$$

und
wobei der Score ein Abstand zwischen genomischen Daten eines individuellen Organismus und genomischen Daten des Rests der Hintergrundpopulation ist;
wobei der lokale Score ein durchschnittlicher euklidischer Abstand von dem mindestens einen Abstand zu dem Cluster ist, der das mindestens eine Subjekt umfasst;
wobei der globale Score ein durchschnittlicher euklidischer Abstand für die Anzahl von individuellen Organismen innerhalb des Clusters zu allen anderen der individuellen Organismen innerhalb der Kohorte ist;
wobei die Kohortengröße die Anzahl von individuellen Organismen in der Kohorte ist; und
wobei die Clustergröße die Anzahl von individuellen Organismen in dem Cluster ist.

**12.** Verfahren nach Anspruch 1, wobei:

das mindestens eine Subjekt und die Hintergrundpopulation eine Kohorte definieren; und
der Bericht eine Score umfasst, bestimmt durch die Formel:

$$Score\ =\ (x\ *\ y\ *\ z)^{\frac{1}{3}}$$

wobei gilt:

$$X = \frac{r^{1+\frac{Rang}{n}}}{r};$$

wobei gilt:

$$Y = \frac{Eigenscore}{s}$$

wobei gilt:

$$Z = \frac{Eigenscore - m1}{m2}$$

wobei gilt:

$$r = e^{150};$$

wobei m1 der Mindest-Score eines beliebigen individuellen Organismus in der Kohorte ist;
wobei m2 der Höchst-Score eines beliebigen individuellen Organismus in der Kohorte ist;
wobei n die Anzahl von individuellen Organismen in der Kohorte ist;
wobei s die Summe aller Eigenscores in der Kohorte ist;
wobei Rang für den Rang der individuellen Organismen innerhalb der Hintergrundpopulation steht; und
wobei der Eigenscore der spektrale Clustering-Score ist.

## Revendications

1. Procédé de prédiction des caractéristiques phénotypiques d'un organisme individuel, comprenant :

l'accès, par un processeur, à des données génomiques d'un certain nombre d'organismes individuels comprenant au moins un sujet et une population de fond ;
l'identification, par le processeur, d'un certain nombre de sites variants dans les données génomiques pour l'au moins un sujet en vue de la population de fond ;
pour chaque site variant identifié :

la récupération, par le processeur, à partir d'un premier répertoire de données par le processeur, d'une force d'effet sur au moins un phénotype associé au site variant identifié ;
la récupération, par le processeur, à partir d'un deuxième répertoire de données par le processeur, d'un degré d'association avec un terme ontologique associé au site variant identifié ; et
la détermination, par le processeur, du fait que le site variant identifié est pertinent lorsqu'une somme pondérée d'un score pour la force d'effet et d'un score pour le degré d'association avec le terme ontologique est supérieure à un seuil ;
pour chaque site variant pertinent :

la formation, par le processeur, d'une matrice pour l'au moins un phénotype, la matrice ayant une pluralité de cellules avec chaque cellule notée pour indiquer une distance comparant le degré de correspondance entre l'au moins un sujet et chaque organisme individuel dans le nombre d'organismes individuels afin de déterminer les distances entre l'au moins un sujet et la population de fond pour l'au moins un phénotype ; et
la génération, par le processeur, d'un rapport pour l'au moins un sujet qui inclut des variants pertinents qui possèdent des différences statistiques par rapport à la population de fond en générant, par le processeur, un score de regroupement spectral par
transformation de la matrice en une matrice de similarité à l'aide d'un noyau gaussien G déterminé par la formule :

$$G(x, \sigma) \; = \; exp \; \left( \frac{-x^2}{\sigma^2} \right)$$

où

$$\sigma \; = \sqrt{max(x^2)} \; ;$$

à partir de la matrice de similarité, la résolution d'un problème propre en obtenant le laplacien pour générer une pluralité de vecteurs propres ;

la sélection des N premiers vecteurs propres les plus petits ; et

l'utilisation des vecteurs propres sélectionnés pour une partition en groupes via un regroupement en K moyens standard.

2. Procédé selon la revendication 1, dans lequel l'au moins un sujet comprend une pluralité de sujets.

3. Procédé selon la revendication 1, dans lequel chaque distance dans la matrice est obtenue en ajoutant toutes les contributions de distribution de chaque variant pertinent à partir d'une entrée de répertoire associée pour les conséquences connues de variants, et dans lequel le répertoire associé est une base de données d'analyse fonctionnelle par le modèle de Markov caché (FATHMM) qui prédit les conséquences fonctionnelles des variants de nucléotides simples non synonymes et des variants non codants.

4. Procédé selon la revendication 1, dans lequel le rapport comprend en outre un indicateur de signification statistique pour l'au moins un sujet par rapport à une pluralité de phénotypes.

5. Procédé selon la revendication 1, dans lequel l'étape de génération du rapport comprend en outre la génération, par le processeur, d'un score de dimensionnalité intrinsèque qui décrit le nombre de variables nécessaires pour représenter le phénotype.

6. Procédé selon la revendication 1, dans lequel le seuil est déterminé, en partie, en tant qu'une variance de seuil indiquant qu'un score de pertinence est une aberration dérivée d'un ensemble de scores de pertinence pour chaque phénotype.

7. Procédé selon la revendication 1, dans lequel l'étape de récupération de la force d'effet sur un phénotype associé au site variant identifié comprend en outre la récupération de la force d'effet à partir d'une base de données d'analyse fonctionnelle par le modèle de Markov caché (FATHMM) qui prédit les conséquences fonctionnelles de variants de nucléotides simples non synonymes et de variants non codants.

8. Procédé selon la réclamation 1,

dans lequel le rapport comprend en outre un score de priorisation de phénotype pour chaque phénotype pour l'au moins un sujet ;

le procédé comprenant en outre :

le classement des scores de priorisation des phénotypes.

9. Procédé selon la revendication 8, comprenant en outre l'extraction de l'identification d'allèles à partir d'un phénotype de rang supérieur dans les entrées classées des phénotypes.

10. Procédé selon la revendication 1, dans lequel la distance est déterminée en utilisant la fréquence de terme fréquence de document inverse, TF-IDF.

11. Procédé selon la revendication 1, dans lequel :

une cohorte comprend l'au moins un sujet et la population de fond ; et

la distance est déterminée par la formule :

$$Score = score\_local + \mu\ score\_global\ ;$$

où, $\mu$ est une correction de la taille d'un groupe pour un groupe déterminé par la formule :

$$\mu\ =\ \frac{e^{\left(\gamma\frac{taille\_cohorte - taille\_groupe}{taille\_cohorte}\right)-1}}{e^{\gamma}-1}\ ;$$

et

dans lequel le score est une distance entre les données génomiques d'un organisme individuel et les données génomiques du reste de la population de fond ;

dans lequel le score local est une distance euclidienne moyenne de l'au moins un sujet du groupe comprenant l'au moins un sujet ;

dans lequel le score global est une distance euclidienne moyenne pour le nombre d'organismes individuels dans le groupe par rapport à tous les autres organismes individuels dans la cohorte ;

dans lequel la taille de la cohorte est le nombre d'organismes individuels dans la cohorte ; et

dans lequel la taille du groupe est le nombre d'organismes individuels dans le groupe.

12. Procédé selon la revendication 1, dans lequel :

l'au moins un sujet et la population de fond définissent une cohorte ; et
le rapport comprend un score est déterminé par la formule :

$$score\ =\ (x\ *\ y\ *\ z)^{\frac{1}{3}}$$

dans lequel :

$$X\ =\ \frac{r^{1+\frac{rang}{n}}}{r}\ ;$$

dans lequel :

$$Y\ =\ \frac{score\ propre}{s}$$

dans lequel :

$$Z\ =\ \frac{score\ propre - m1}{m2}$$

dans lequel :

$$r\ =\ e^{150}\ ;$$

dans lequel m1 est le score minimum d'un quelconque organisme individuel dans la cohorte ;
dans lequel m2 est le score maximum d'un quelconque organisme individuel dans la cohorte ;
dans lequel n est le nombre d'organismes individuels dans la cohorte ;
dans lequel s est la somme de tous les scores propres dans la cohorte ;
dans lequel rang est le rang de l'organisme individuel dans la population de fond ; et
dans lequel le score propre est le score de regroupement spectral.

*FIG. 1A*

**FIG. 1B**

A2

112A    112B    112C    112D

Variant 1    Variant 2    Variant 3    Variant 4

| Strong (7) Lactase | Strong (9) Maltase | Weak (0) Amylase | Weak (1) Sucrase |

118A    118B    118C    118D

Determine presence of associated Ontological term (e.g., dcGO)

B2

**FIG. 1C**

| Variant 1 | Variant 2 | Variant 3 | Variant 4 |
|---|---|---|---|
| Strong (7) 114A | Weak (3) 114B | Strong (8) 114C | Weak (1) 114D |
| Strong (7) 118A | Strong (9) 118B | Weak (0) 118C | Weak (1) 118D |
| Relevant | Relevant | Not Relevant | Not Relevant |

*FIG. 1D*

FIG. 1E

200

| ontology terms | | score | rank in cohort | eigenscore | Idimension | Rank in cohort (by mean distance) | Number of Variants | Ontology definition |
|---|---|---|---|---|---|---|---|---|
| HP:0003115 | Abnormal EKG | 0.00124 | 5 | 2.6779 | 1.27895 | 4 | 9 | |
| HP:0001250 | Seizures | 0.00058 | 4 | 1.81424 | 1.54686 | 66 | 32 | "Seizures are an intermittent `abnormality of the central nervous system` (FMA:HP:0002011) due to a sudden, excessive, disorderly discharge of cerebral neurons and characterized clinically by some combination of disturbance of sensation, loss of consciousness, impairment of psychic function, or convulsive movements. The term epilepsy is used to describe chronic, recurrent seizures." [HPO:probinson] |
| HP:0011804 | Abnormality of muscle physiology | -0.00212 | 10 | 3.91218 | 3.59253 | 25 | 211 | "A functional abnormality of a skeletal muscle." [HPO:probinson] |
| HP:0001373 | Joint dislocation | -0.00537 | 12 | 2.8857 | 8.40083 | 180 | 32 | "Displacement or malalignment of joints." [HPO:curators] |
| HP:0003272 | Abnormality of the hip bone | | | | | | | "An abnormality of the `hip bone` (FMA:16585)." [HPO:probinson] |
| HP:0001384 | Abnormality of the hip joint | -0.00588 | 13 | 3.14139 | 6.60912 | 157 | 40 | "An abnormality of the `hip joint` (FMA:35178)." [HPO:probinson] |
| MP:0005193 | abnormal anterior eye segment morphology | -0.00904 | 18 | 3.55774 | 3.717 | 19 | 122 | "any structural anomaly of any of the parts of the eye that lie in front of, or ventral to, the lens (inclusive)" [ISBN:0-683-40008-8, MESH:A09.371.060] |

202  204  206  208  210  212  214  216

218

*FIG. 2*

| D2P2 SNPedia ENSEMBL 1000 Genomes | allele | allele freq.s | variant weight | ave. distance from cohort | other phenotypes \| score \| name | | |
|---|---|---|---|---|---|---|---|
| D2P2 SNPedia 16_57949224_G/T rs10459809 | TT | G:0.8942 T:0.1057 | 0.57 | 0.160 | MP:0003492 | 8.142 | abnormal involuntary movement |
| | | | | | MP:0002066 | 3.536 | abnormal motor capabilities/coordination/movement |
| | | | | | DOID:1826 | 3.490 | epilepsy syndrome |
| | | | | | MP:0001970 | 3.475 | abnormal pain threshold |
| | | | | | DOID:2234 | 3.466 | focal epilepsy |
| | | | | | MP:0003216 | 3.345 | absence seizures |
| | | | | | MP:0002206 | 3.159 | abnormal CNS synaptic transmission |
| | | | | | MP:0002064 | 3.154 | seizures |
| | | | | | HP:0003115 | 2.677 | Abnormal EKG |
| | | | | | HP:0001250 | 1.814 | Seizures |
| D2P2 SNPedia 2_233633460_G/A rs1801251 | AA | G:0.7129 A:0.2870 | 0.51 | 0.121 | MP:0003492 | 8.142 | abnormal involuntary movement |
| | | | | | HP:0001832 | 4.324 | Abnormality of the metatarsal bones |
| | | | | | MP:0002184 | 3.696 | abnormal innervation |
| | | | | | MP:0002066 | 3.536 | abnormal motor capabilities/coordination/movement |
| | | | | | DOID:1826 | 3.490 | epilepsy syndrome |
| | | | | | MP:0001970 | 3.475 | abnormal pain threshold |
| | | | | | DOID:2234 | 3.466 | focal epilepsy |
| | | | | | MP:0000968 | 3.369 | abnormal sensory neuron innervation pattern |
| | | | | | MP:0003216 | 3.345 | absence seizures |
| | | | | | MP:0002206 | 3.159 | abnormal CNS synaptic transmission |
| | | | | | MP:0002064 | 3.154 | seizures |
| | | | | | HP:0003115 | 2.677 | Abnormal EKG |
| | | | | | MP:0002144 | 2.379 | abnormal B cell differentiation |
| | | | | | HP:0001250 | 1.814 | Seizures |
| | | | | | MP:0002367 | 1.621 | abnormal thymus lobule morphology |

FIG. 3A

sequences for this phenotype
(ID | substitution | superfamily | E-value | family | E-value)

| ID | substitution | superfamily | E-value | family | E-value |
|---|---|---|---|---|---|
| ENSP00000454633 | L739I | Voltage-gated potassium channels | 3.01e-24 | Voltage-gated potassium channels | 0.0046 |
| ENSP00000251102 | L745I | | 3.14e-24 | | 0.0046 |
| ENSP00000386251 | T175I | | 4.57e-54 | | 0.00024 |
| ENSP00000386251 | T175I | | 3.27e-25 | | 0.002 |
| ENSP00000407284 | T95I | E set domains | 1.07e-31 | Cytoplasmic domain of inward rectifier potassium channel | 0.0019 |
| ENSP00000233826 | T175I | | 4.57e-54 | | 0.00024 |
| ENSP00000233826 | T175I | | 3.27e-25 | | 0.002 |

FIG. 3B

| 304 | 306 | 308 | 310 | 312 | 314 | | |
|---|---|---|---|---|---|---|---|
| **302C** D2P2 SNPedia 1_26520292_G/T rs12138368 | GT | G:0.9661 T:0.0338 | 0.67 | 0.098 | MP:0003492 | 8.142 | abnormal involuntary movement |
| | | | | | MP:0002066 | 3.536 | abnormal motor capabilities/coordination/movement |
| | | | | | DOID:1826 | 3.490 | epilepsy syndrome |
| | | | | | MP:0001970 | 3.475 | abnormal pain threshold |
| | | | | | DOID:2234 | 3.466 | focal epilepsy |
| | | | | | MP:0003216 | 3.345 | absence seizures |
| | | | | | MP:0002206 | 3.159 | abnormal CNS synaptic transmission |
| | | | | | MP:0002064 | 3.154 | seizures |
| | | | | | HP:0003115 | 2.677 | Abnormal EKG |
| | | | | | HP:0001250 | 1.814 | Seizures |
| **302D** D2P2 SNPedia 8_87679303_A/C rs6471482 | CC | A:0.0682 C:0.9317 | 0.58 | 0.034 | MP:0003492 | 8.142 | abnormal involuntary movement |
| | | | | | MP:0002066 | 3.536 | abnormal motor capabilities/coordination/movement |
| | | | | | DOID:1826 | 3.490 | epilepsy syndrome |
| | | | | | MP:0001970 | 3.475 | abnormal pain threshold |
| | | | | | DOID:2234 | 3.466 | focal epilepsy |
| | | | | | MP:0003216 | 3.345 | absence seizures |
| | | | | | MP:0002206 | 3.159 | abnormal CNS synaptic transmission |
| | | | | | MP:0002064 | 3.154 | seizures |

300

*FIG. 3C*

| | | | | | |
|---|---|---|---|---|---|
| ENSP00000341006 | L124F | | 1.1e-24 | | 0.014 |
| ENSP00000390423 | L124F | Voltage-gated potassium channels | 7.22e-25 | Voltage-gated potassium channels | 0.014 |
| ENSP00000429464 | L124F | | 5.1e-20 | | 0.01 |
| ENSP00000316605 | C234W | Voltage-gated potassium channels | 4.84e-27 | Voltage-gated potassium channels | 0.0059 |

*FIG. 3D*

| Rank | Individual | Relative Score | Absolute Score | Mean Distance |
|---|---|---|---|---|
| 1 | 860 | 0.005094 | 2.78409 | 13.72926 |
| 2 | 436 | 0.004046 | 2.71134 | 7.56593 |
| 3 | 901 | 0.003098 | 2.69329 | 10.26487 |
| 4 | 1012 | 0.002178 | 2.69032 | 6.73771 |
| 5 | 0 | 0.00124 | 2.6779 | 11.51051 |
| 6 | 686 | 0.00031 | 2.66944 | 11.65165 |
| 7 | 308 | -0.00336 | 2.66588 | 6.0304 |
| 8 | 1036 | -0.00336 | 2.66588 | 6.0304 |
| 9 | 252 | -0.00336 | 2.66588 | 6.0304 |
| 10 | 316 | -0.00336 | 2.66588 | 6.0304 |
| 11 | 375 | -0.00703 | 2.65833 | 6.32716 |
| 12 | 139 | -0.00703 | 2.65833 | 6.32716 |
| 13 | 777 | -0.00703 | 2.65833 | 6.32716 |
| 14 | 448 | -0.00703 | 2.65833 | 6.32716 |
| 15 | 717 | -0.00978 | 2.65527 | 6.88596 |
| 16 | 85 | -0.00978 | 2.65527 | 6.88596 |
| 17 | 1016 | -0.00978 | 2.65527 | 6.88596 |
| 18 | 961 | -0.01161 | 2.65297 | 7.83072 |
| 19 | 204 | -0.01161 | 2.65297 | 7.83072 |
| 20 | 71 | -0.01622 | 2.63539 | 6.56461 |
| 21 | 550 | -0.01622 | 2.63539 | 6.56461 |
| 22 | 429 | -0.01622 | 2.63539 | 6.56461 |

*FIG. 4*

500

502A  502B  502C  502D

504

Genome
Data

Strength of effect
on the phenotype
(e.g., FATHMM)

506

510

512

Reporting

508

Determine presence of
associated Ontological term
(e.g., dcGO)

514

Diagnostic/
Therapy

*FIG. 5*

*FIG. 6*

EP 3 405 897 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHIHAB, H.A. ; GOUGH, J. ; COOPER, D.N. ; BARKER, G.L.A. ; EDWARDS, K.J. ; DAY, I.N.M. ; GAUNT, T.** Predicting the Functional, Molecular, and Phenotypic Consequences of Amino Acid Substitutions using Hidden Markov Models. *Human mutation,* 2012, vol. 34 (1), 57-65
- **FANG et al.** dcGO: database of domain-centric ontologies on functions, phenotypes, diseases and more. *Nucleic acids research,* 17 November 2012, vol. 41 (D1
- **LIU et al.** Analysis of correlated mutations in HIV-1 protease using spectral clustering. *Bioinformatics,* 28 March 2008, vol. 24 (10
- **SHIBAB et al.** Predicting the Functional, Molecular, and Phenotypic Consequences of Amino Acid Substitutions using Hidden Markov Models. *Human Mutation,* 2012, vol. 34 (1

- **JONATHAN VON BRÜNKEN ; MICHAEL E. HOULE ; ARTHUR ZIMEK.** Intrinsic Dimensional Outlier Detection in High-Dimensional Data. National Institute of Informatics
- *Human Phenotype, http://human-phenotype-ontology.github.io*
- *Mammalian Phenotype, http://www.informatics.jax.org/searches/MP_form.shtml*
- *Gene Ontology, http://amigo.geneontology.org*
- *Drugbank, http://www.drugbank.ca*
- **PHAM, DUC TRUONG ; STEFAN S. DIMOV ; C. D. NGUYEN.** Selection of K in K-means clustering. *Proceedings of the Institution of Mechanical Engineers, Part C: Journal of Mechanical Engineering Science,* 2005, vol. 219 (1), 103-119